# EUROPEAN PATENT APPLICATION

(11) **EP 1 834 960 A2**
(43) Date of publication of application: **19.09.2007**
(21) Application number: 07011281.8
(22) Date of filing: 04.12.2001
(51) Int. Cl.: C07K 14/065, C12N 15/39, A61P 37/00, A61K 48/00, A61K 38/16, G01N 33/68

(54) **Immunomodulatory protein derived from the yaba monkey tumor virus**

(30) Priority: 04.12.2000 US 251147 P
(62) Divisional of application: 01999573.7
(71) Applicant: Viron Therapeutics, Inc., London, Ontario N6G 4X8 (CA)
(72) Inventor: The designation of the inventor has not yet been filed
(74) Representative: Bösl, Raphael Konrad

(57) **Abstract**

This invention features a therapeutic composition for the treatment of an inflammatory or immune-mediated disease condition, the therapeutic composition comprising an isolated Yatapoxvirus cytokine inhibitor protein formulated in a pharmaceutically acceptable vehicle, a method of modulating cytokine function in a cell in vitro, said method comprising contacting said cell with an isolated Yatapoxvirus cytokine inhibitor protein and the use of an isolated Yatapoxvirus cytokine inhibitor protein for the manufacture of a medicament for the treatment of an inflammatory or immune-mediated disease condition.

## Description

### Field of the Invention

This invention relates to a novel secreted viral protein that may be utilized in the treatment of human diseases. The protein disclosed herein is isolated from the genome of the poxvirus family, where said virus is the Yaba Monkey Tumor Virus. This invention reveals the non-obvious discovery of the sequence of this novel immunomodulatory protein and useful embodiments thereof that may mediate an anti-inflammatory and/or immunomodulatory role when administered in vivo.

### Background of the invention

Poxviruses are a large family of DNA viruses known to infect a variety of mammalian species. To date, approximately 50 poxvirus genomes have been identified and each genome contains about 200 open reading frames encoded therein. The poxvirus family, otherwise known as Poxviridae, includes two subfamilies (Chordopoxvirinae and Entomopoxvirinae) wherein the species are divided into eight and three genuses respectively, including but limited to Orthopoxvirus, Parapoxvirus, Avipoxvirus, Capripoxvirus, Leporipoxvirus, Suipoxvirus, Molluscipoxvirus and Yatapoxvirus, which include but are not limited to the species known as Myxoma Virus, Vaccinia Virus, Swinepox Virus, Molluscum Contagiosum Virus and Yaba Monkey Tumor Virus. Poxviruses are characterized as large, brick-like virions with complex symmetry that share antigenic determinants among different species of the family.

It is well known within the art that, upon infection of a host organism, the poxvirus genome mediates expression of numerous proteins that interfere and modulate homeostasis within the host. In addition to proteins that mediate an intracellular effect, poxviruses are also known to secrete proteins into the circulatory system of the infected animal. Such secreted proteins include agents that bind and inhibit various different aspects of the mammalian immune system and minimize immune-mediated clearance of the virus.

The Yaba Monkey Tumor Virus (YMTV) is poxvirus of the Yatavirus genus and was characterized in 1958 during outbreaks of rhesus monkeys. YMTV infection in monkeys leads to epidermal histiocytomas that advance to suppurative inflammatory reactions. YMTV has a DNA genome of 136 kilobases with a large part of the genome yet to be cloned and characterized. YMTV grows relatively slowly in primate cell culture lines and its host range is restricted to a small number of primates, and occasionally man following accidental exposure to infected monkeys.

IL-18 is a pro-inflammatory mammalian cytokine that plays an important early function in the potentiation of Tₕ1-like immune responses. In addition to its independent effects, IL-18 synergizes with IL-12 to induce IFN- production from various immune cell types. Binding of IL-18 to specific cell-surface receptors induces NF- B activation and IL-18 is important in vivo for production of IFN- and inflammatory responses that may contribute to inflammatory disease. These diseases include but are not limited to allergic inflammation, atherosclerotic plaque growth and unstable plaque rupture, arterial restenosis, by-pass graft occlusion, Gaucher's disease, diabetes mellitus, rheumatoid arthritis, multiple sclerosis, transplant rejection, transplant vasculopathy and glomerulonephritis.

This invention provides the non-obvious identification and characterization of a protein derived from YMTV, where said protein is referred to as YMTV Cytokine Inhibitor, or YCI. The invention embodies nucleic acid and amino acid polymers encoding said YCI, including methods of detecting and producing said YCI. The invention further embodies the use of this YCI as a method of modulating the immune response within an organism. It is further claimed herein that the nucleic acid and amino acid sequences disclosed may be utilized for the treatment of human individuals for the purpose of preventing, treating or reversing the onset of one or more immune-related diseases, including inflammation and the immune-mediated diseases outlined above, but not limited to such.

### Brief Description of the Drawings

The present invention will be further understood and supported using the following drawings and their associated descriptions. These descriptions and drawings are not meant to limit the invention by any circumstance and are to be interpreted as possible embodiments of the invention disclosed herein. The methods utilized in the generation of the data represented by these drawings are commonly known within the art and may be reproduced identically by employing the methods described herein.

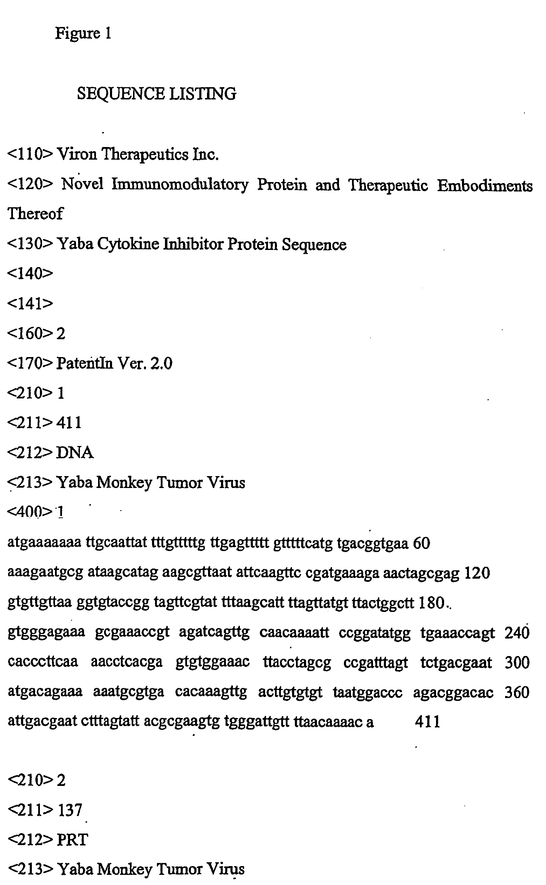

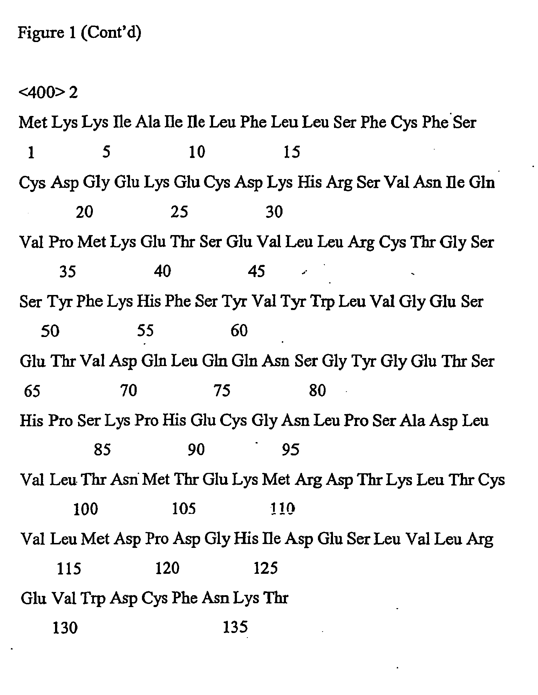

Figure 1 illustrates the genomic nucleic acid sequence of the YCI gene within YMTV, hereinafter referred to as "SEQ ID NO:1" and further illustrates the amino acid sequence of YCI protein expressed by YMTV, hereinafter referred to as "SEQ ID NO:2".

### Summary of the Invention

The invention disclosed herein characterizes a novel nucleic acid and protein sequence derived from the Yaba Monkey Tumor Virus (YMTV). The invention embodies all compositions associated with said sequences, including but not restricted to DNA and RNA fragments derived thereof, as well as amino acid sequences derived of the disclosed sequence. It is contemplated herein that the disclosed protein sequence, as well as homologs, analogs and fragments thereof, are capable of binding aspects of the mammalian immune system with detectable affinity and stability. In one such embodiment, such binding occurs between the disclosed protein and one or more mammalian cytokines, where one such cytokine is interleukin-18, or IL- 18 as known within the art. The disclosed protein is therefore herein referred to as YMTV Cytokine Inhibitor, or YCI. The invention further embodies use of said YCI nucleic acid, including complimentary sequences thereto, and protein sequences for a variety of diagnostic and therapeutic applications, in one such embodiment, it is contemplated herein that YCI sequences may be utilized to identify interactions with immune related proteins. In a further embodiment, the YCI sequences disclosed herein may be introduced into a mammalian organism for the purpose of modulating the incidence, progression and pathogenesis of an inflammatory, auto-immune or immune-mediated disease condition within said organism.

### Detailed Description of the Invention

The invention disclosed hereinunder identifies a novel protein derived from the Yaba Monkey Tumor Virus, which is well known within the art as a member of the poxvirus family and a further subset of the Yatapoxvirus genus therein. Said Yaba Monkey Tumor Virus is hereinafter referred to as "YMTV". In particular, this invention discloses an immunomodulatory nucleic acid and amino acid sequence relating thereto, referred to as the Yaba Cytoldne Inhibitor, where the nucleic acid sequence and amino acid sequences, including homologs, analogs and truncations thereof, are hereinafter collectively abbreviated as "YCI". The disclosure herein contains relevant nucleic acid and protein sequences of YCI within SEQ ID NO:1 and SEQ ID NO:2, respectively, as attached hereinafter. Thus, the YCI gene has a length of 411 nucleotides, corresponding to an amino acid sequence of 137 amino acids encoded therein.

The scope of this invention includes variations of the nucleic acid sequences defined as follows:
(1) truncations, analogs and homologs of the nucleic acid sequences provided in SEQ ID NO:1;
(2) nucleic acid sequences differing from SEQ ID NO: by substitution of a T with a U;
(3) nucleic acid sequences complimentary to either the full length of SEQ ID NO: 1, or complimentary to nucleic acid fragments derived thereof;
(4) nucleic acid sequences that hybridize with the nucleic acid sequences represented within SEQ ID NO:1;
(5) nucleic acid sequences differing from the full length of

SEQ ID NO:1 due to the degeneracy of the genetic code.

The term "nucleic acid" is intended to include DNA and RNA that can either be of single or double stranded structure. The term "protein" or "polypeptide" refers to amino acid polymers existing in an unfolded or folded spatial organization and with or without catalytic function. The term "antibody" refers to protein molecules derived from a polyclonal or monoclonal population of B cells of mammalian origin. The term "antibody fragment" refers to the aforementioned antibody molecules that have been cleaved into different segments and/or may be labeled with fluorochrome compounds for the purpose of detection. The term "chemokine" refers to all known chemotactic cytokines expressed within mammalian organisms that mediate the recruitment and infiltration of leukocytes into tissues. The term "chemokine" includes but is not limited to all mammalian members of the C, CC, CXC, and CXXXC families of chemotactic cytokines, classified within the art based upon the distribution of cystine residues therein. The term "chemokine receptor" refers to all known transmembrane proteins known within the art to interact with one or more chemokines. The term "chemokine receptor" shall include but is not limited to all chemokine receptors classified within the art as CR, CCR, CXCR and CXXXCR The term "cytokine" refers to all human cytokines known within the art that bind extracellular receptors upon the cell surface and thereby modulate cell function, including but not limited to IL-1, IL-4, IL-6, IL-18, TNF- and IFN- . The term "cytokine receptor" refers to all human cytokine receptors within the art that bind one or more cytokine(s), as defined hereinunder, including but not limited to receptors of IL-1, IL-4, IL-6, IL-18, TNF- and IFN-

The following standard abbreviations are utilized throughout specification of the present invention and its included drawings: DNA - deoxyribonucleic acid; RNA - ribonucleic acid; C - cytosine; G - guanine; A - adenosine; T- thymidine; N - unknown; A, Ala - alanine; C, Cys - cysteine; D, Asp - aspartic acid; E, Glu - glutamic acid; F, Phe - phenylalanine; G, Gly - glycine; H, His - histidine; I, Ile - isoleucine; K, Lys - lysine; L, Leu - leucine; M, Met - methionine; N, Asn - asparagine; P, Pro - proline; Q, Gln - glutamine; R, Arg - arginine; S, Ser - serine; T, Thr - threonine; V, Val - valine; W, Trp - tryptophan; Y,Tyr - tyrosine; and pY, pTyr - phosphotyrosine.

In one embodiment, this invention contemplates a purified or isolated double stranded nucleic acid molecule formed through hydrogen bonding of the nucleic acid molecules specified in SEQ ID NO:1, or related truncations, homologs and analogs thereof, to a complimentary nucleic acid sequence.

The nucleic acid molecules specified herein as SEQ ID NO:1 may also be inserted into an expression vector that contains necessary elements upstream and downstream of the inserted nucleic acid for the transcription and translation of the inserted sequence within prokaryotic and eukaryotic cells. The invention embodies expression vectors which comprise a nucleic acid molecule specified in SEQ ID NO:1, or related truncations, homologs and analogs thereof, with one or more transcription and translation elements operatively linked to the nucleic acid molecule. Possible expression vectors include, but are not limited to, cosmids, plasmids and modified viral vectors (replication-defective retroviruses, adenoviruses and adeno-associated viruses).

Recombinant expression vectors may be used to prepare transformed cell lines expressing the proteins encoded within nucleic acid sequences specified herein by SEQ ID NO:1, or related truncations, homologs and analogs thereof. This invention provides cell lines, including eukaryotic and prokaryotic cell types, containing a recombinant nucleic acid molecule specified herein within SEQ ID NO:1, or related truncations, homologs and analogs thereof.

This invention also contemplates transgenic non-human animals whose germ cells and somatic cells contain a recombinant molecule comprising a nucleic acid molecule specified in SEQ ID NO:1, or a related truncation, analog or homolog thereof. Such sequences may be expressed in non-human species including but not limited to zebrafish, xenopus, drosophila, mice, rats, rabbits, sheep, pigs and chickens.

This invention also embodies YCI encoded by SEQ ID NO:2, or related homologs, truncations and analogs thereof. This invention embodies all post-translational modifications of YCI and related homologs, truncations and analogs thereof. Such post-translational modifications include, but are not limited to, glycosylation, myristylation, tyrosine phosphorylation, serine phosphorylation, threonine phosphorylation, ubiquitination and proteolytic degradation.

This invention also embodies a method of preparing YCI encoded by SEQ ID NO:2, or related truncations, analogs and homologs thereof. A method of preparing such protein molecules is embodied by (1) transferring a recombinant expression vector of the herein specified YCI encoded by SEQ ID NO:1, (2) selecting transformed host cells from untransformed cells, (3) culturing the host cell under conditions that allow or induce the expression of the specified YCI encoded by SEQ ID NO:1 and (4) isolation of YCI from cultured host cells using a suitable purification procedure. In such embodiments, YCI may be produced within either prokaryotic or eukaryotic host cells, as appropriate.

This invention also embodies the purification of YCI encoded by SEQ ID NO:2, or related truncations, analogs and homologs thereof. In one embodiment, physical and chemical characteristics of YCI as specified herein are utilized to separate said protein from other protein or non-protein molecules. Such physical and chemical characteristics include but are not limited to, density, molecular weight, isoelectric point, ligand affinity, solubility, temperature-sensitivity, etc.

This invention also contemplates the conjugation of YCI encoded by SEQ ID NO:2, or related truncations, analogs and homologs thereof, to other protein or non-protein molecules. This may be accomplished by covalent attachment of conjugating molecules to any residue of the specified YCI encoded by SEQ ID NO:2, or related truncations, analogs and homologs thereof.

This invention also contemplates antibodies or antibody-derived fragments specifically capable of binding the specified YCI encoded by SEQ ID NO:2 or any segment thereof. Therefore, this invention also provides a method of generating antibodies within mammalian species through injection of the specified YCI encoded by SEQ ID NO:2 or segments of the amino acid sequence thereof into a mammalian organism.

Furthermore, antibodies or antibody fragments specific for the specified YCI encoded by SEQ ID NO:2 or segments thereof may be labeled with detectable substances, such as fluorochromes or peroxidases, that permit detection of the YCI, or related sequences specified within SEQ ID NO:2, within tissues and cells. The invention also covers use of such antibodies to purify YCI or its related homologs, analogs and truncations from cells and tissues.

This invention also provides a method for the design and construction of nucleotide probes unique to nucleic acid molecules encoding YCI encoded by SEQ ID NO:1 or variations thereof. Such nucleotide probes may also be labeled with detectable substances that permit detection of YCI encoding nucleic acid sequences within tissues and cells. In addition, nucleotide probes may also be utilized as a diagnostic tool to assess the upregulation of YCI expression within cells. Labeled nucleotide probes may alternatively be used to identify YCI related nucleic acid molecules from a heterogeneous population of deoxyribonucleic acids and/or ribonucleic acids (e.g., a cDNA library, a genomic DNA library or a genomic RNA library).

This invention also embodies the use of the polymerase chain reaction or related polymerase reactions to amplify or generate nucleic acids encoding YCI, including related truncations, analogs or truncations thereof. In one embodiment, synthetic oligonucleotide primers generated from segments of the nucleotide sequence disclosed in SEQ ID NO: 1 can be utilized to amplify YCI-encoding sequence(s) from genomic DNA, cDNA libraries, RNA molecules or other nucleic acid mixtures.

This invention also provides a method of modulating YCI expression within cells, tissues, organs and organisms. The introduction of nucleic acid molecules into cells and tissues may be utilized to amplify the transcription and translation of YCI-encoding nucleic acids or related truncations, analogs or homologs or related nucleic acid sequences as specified by SEQ ID NO: 1. Alternatively, YCI expression may be downregulated by the introduction of complimentary nucleic acid sequences that block transcription and translation of YCI-encoding nucleic acids or related truncations, analogs or homologs or related nucleic acid sequences as specified by SEQ ID NO:1.

In a preferred embodiment, YCI encoded herein as SEQ ID NO:2 may bind one or more types of chemoldnes derived from or present within mammalian organisms. Therefore, YCI may be administered in vivo to bind one or more chemokine proteins within mammalian organisms. Furthermore, YCI may be administered or expressed within specific mammalian tissues to bind chemokines present within said tissue. It is envisioned herein that the binding between YCI and said chemokine shall reduce, inhibit and/or otherwise diminish the ability of said chemokine to conduct its normal function within mammalian organisms. In one embodiment, the binding between YCI and one or more chemokine(s) shall occur in the region of the chemokine molecule responsible for interaction with a corresponding receptor thereof, hence preventing the covalent or non-covalent interaction between said chemokine and its corresponding chemokine receptor.

In another preferred embodiment, YCI encoded herein as SEQ ID NO:2 may bind mammalian cytokines, other than those defined herein as chemokines. As such, YCI may be administered in vivo to bind one or more types of cytokines within mammalian organisms. Alternatively, YCI may be administered or expressed within specific mammalian tissues to bind cytokines, other than those herein defined as chemokines, within said tissue. It is envisioned herein that the binding between YCI and said cytokine shall reduce, inhibit and/or other diminish the ability of said cytokine to conduct its normal function within mammalian organisms. In one embodiment, the binding between YCI and one or more cytokine(s) shall occur in the region of the cytokine molecule responsible for interaction with a corresponding receptor thereof, hence preventing the covalent or non-covalent interaction between said cytokine and its corresponding cytokine receptor.

In another preferred embodiment, YCI encoded herein as SEQ ID NO:2 may bind mammalian chemokine receptors, as defined above. Such binding between YCI and one or more chemokine receptors may occur at the extracellular domain of said receptor. Furthermore, it is envisioned herein that such binding between YCI and one or more of the said chemokine receptors may disrupt normal signaling known within the art to occur upon engagement of said receptor with the appropriate chemokine ligand. As such, YCI encoded herein as SEQ ID NO:2 may be administered in vivo to bind one or more chemokine receptors and reduce, inhibit and/or other diminish the signaling functions mediated by such chemokine receptors.

This invention further provides a method for the identification of substances capable of binding YCI encoded herein as SEQ ID NO:2, or related truncations, analogs or homologs derived from viral, bacterial or mammalian sources. In one embodiment, YCI is present in proximity to other proteins derived from mammalian sources, including the appropriate conditions necessary for binding to occur, while binding is detected using YCI-specific labeled antibodies. In another embodiment, a yeast two hybrid assay system is utilized as a method for the identification of proteins interacting with YCI, its truncations, analogs or homologs derived from mammalian sources. In another embodiment, interactions between YCI and other proteins, including but not restricted to immune-related proteins, are detected through cross-linking agents as are known within the art that mediate covalent bonds between YCI and other proteins that demonstrate significant affinity thereto.

This invention further provides a method of identifying agents that affect the transcription and or translation of YCI encoding nucleic acids, including but not limited to those represented as SEQ ID NO:1 within cells expressing said protein, including truncations, analogs and homologs. In one embodiment, the pattern and level of YCI encoding RNA and full-length protein is assayed upon treatment of YCI expressing cells under assay conditions including, but not limited to, the treatment of YCI expressing cells with growth factors, hormones, cytokines, phorbol esters, hemagglutinins, antibodies and antibody fragments.

The invention herein also provides a method of identifying agents that modulate post-translational modification of YCI, including truncations, analogs and homologs. Such modifications may play a role in YCI protein functions, cytokine protein functions, chemokine protein functions, chemokine receptor functions and other functions or dysfunctions derived thereof. Examples of such modifications include, but are not limited to, protein folding, disulfide linkage, glycosylation, myristylation, palmitoylation, tyrosine phosphorylation, serine phosphorylation, threonine phosphorylation, ubiquitination and proteolytic degradation.

This invention also provides methods for the generation of experimental models for the study of YCI-encoding nucleic acid and protein functions in vivo or in vitro conditions. Cells, tissues and non-human animals that express, over-express or underexpress YCI-encoding nucleic acids, YCI or any related truncations, analogs or homologs thereof, can be established according to the embodiments of the invention herein. In particular, the generation of transgenic non-human animals may be accomplished via nuclear oocyte microinjection of YCI-encoding nucleic acids will provide novel models for the determination of YCI structure and function. This invention also permits the use of YCI-encoding nucleic acids to develop cell lines to study the effect of YCI expression, over-expression or under-expression in various developmental systems, including, but not limited to, hematopoesis, neurogenesis, mammary development and lung epithelial development, cell homeostasis, cell signaling, cell death, differentiation and neuronal development.

In addition, this invention contemplates human therapeutic uses derived from the herein disclosed YCI-encoding nucleic acids. In a preferred embodiment, the aforementioned methods are utilized to reduce, treat, prevent or otherwise lower human conditions associated or mediated by inflammation. In one preferred embodiment, the herein disclosed YCI-encoding nucleic acids are introduced into a mammalian animal through methods and procedures well known within the art. In such situations, it is envisioned that such nucleic acids shall be introduced into cells and tissues that shall mediate replication, transcription and/or translation of said nucleic acids. In another preferred embodiment, YCI-encoding nucleic acids are into specific tissue or cell type of a mammalian animal for the treatment, prevention and/or reduction of disease conditions associated with inflammation. In yet another preferred embodiment, YCI-encoding nucleic acids are introduced into cells and tissues while in vitro or ex vivo conditions, that shall mediate replication, transcription and/or transplantation of said nucleic acids, prior to the transplantation of such YCI-expressing cells and tissues into a mammalian organism for the purpose of reducing, treating, preventing and otherwise lowering disease conditions associated or mediated by inflammation.

The invention disclosed herein further contemplates human therapeutic uses derived from the herein disclosed YCI and/or modifications of the same through other embodiments described herein. In a preferred embodiment, the YCI disclosed herein, and related truncations, analogs and homologs thereof, are introduced into a mammalian organism for the purpose of treating, preventing, reducing or otherwise lowering disease conditions associated or mediated by inflammation. In one preferred embodiment, YCI disclosed herein, and related truncations, analogs and homologs thereof, are specifically introduced in vivo within a specific tissue type that is known within the art to be the site or location of a disease conditions associated or mediated by inflammation. In another embodiment, the YCI disclosed herein, and related truncations, analogs and homologs thereof, are introduced into cells and/or a tissue while in vitro or ex vivo conditions, prior to the transplantation of said cells and/or a tissue into a mammalian organism for the purpose of treating, preventing, reducing or otherwise lowering disease conditions associated or mediated by inflammation.

Further envisioned within the scope of this invention is the usage of the invention, its associated nucleic acids, proteins, antibodies, conjugates, analogs, homologs and truncations thereof, and of its embodiment for the treatment of all human diseases and/or conditions that are mediated or associated with the onset of inflammation, as well as human diseases and/or conditions that are mediated or associated with autoimmunity. Such diseases and/or conditions include but are not restricted to inflammation, autoimmune disease and immune-mediated disorders, which include but are not restricted to, allergic inflammation, arterial restenosis, by-pass graft occlusion, Gaucher's disease, diabetes mellitus, rheumatoid arthritis, multiple sclerosis, transplant rejection, transplant vasculopathy and glomerulonephritis.

This invention also contemplates that reagents suitable for the therapies and diagnostics outlined herein may be administered using pharmaceutically acceptable vehicles. Such vehicles include, but are not limited to, expression vectors, microinjection, liposome delivery, subcutaneous injection, intravenous injection, oral administration, inhalation, transdermal application or rectal administration. Such vehicles and related therapeutic regima may be optimized for according to factors such as disease stage, age, sex and weight of the individual. In one embodiment, reagents suitable for the therapies and diagnostics outlined herein may be packaged into convenient kits providing the necessary materials packaged into suitable containers. Such kits may include suitable supports useful and assisting in performing the therapeutic and diagnostic strategies outlined herein.

Other objects, features and advantages of the present invention that become clear as a result of the methods provided herein and depicted in the enclosed drawings are included in this invention. It should be understood that examples and preferred embodiments of the invention herein are given by way of illustration and various alterations and modifications within the spirit of the invention are included as part of the invention herein. Those skilled in the art will recognize alterations and modifications of the invention herein that must however be respected as a part of the present invention.

The following pages 17 to 24 contain specific embodiments.
1. A purified nucleic acid molecule derived from the Yaba Monkey Tumor Virus, as defined by the following, including a fragment, homolog and analog thereof: (i) SEQ ID NO:1.
2. A nucleic acid molecule as in 1, comprising a sequence complimentary to the following, or a fragment, analog and homolog thereof: (i) SEQ ID NO:1.
3. A nucleic acid molecule comprising a sequence as in 1, where each T may be replaced with a U.
4. A recombinant nucleic acid vector containing one or more nucleic acid sequences encoding the nucleic acid sequences of 1.
5. A host cell containing a nucleic acid sequence as in 1, or a fragment, analog and homolog thereof.
6. As in 5, where the host cell is a eukaryotic cell.
7. As in 5, where the host cell is a prokaryotic cell.
8. A protein molecule encoded by the nucleic acid sequences as in 1, or fragments, analogs and homologs thereof, or as encoded by SEQ ID NO:2.
9. An antibody molecule, or fragment thereof, having specificity against an epitope of the protein molecules in 8.
10. As in 8, where said protein in conjugated with a protein or non-protein based moiety.
11. As in 9, where the antibody is labeled with protein and/or non-protein moieties.
12.A method of modulating chemokine function through expression of one or more nucleic acid sequences as in 1.
13. A method of modulating chemokine function through interaction between said proteins in 8, wherein said proteins non-covalently bind one or more mammalian chemokine(s).
14. A method uf modulating cytokine function through expression of one or more nucleic acid sequences as in 1.
15. A method of modulating cytokine function through interaction between said proteins in 8, wherein said proteins non-covalently bind one or more mammalian cytokine(s).
16. A method of modulating chemokine receptor function through expression of one or more nucleic acid sequences as in 1.
17. A method of modulating chemokine receptor function through interaction between said proteins in 8, wherein said proteins non-covalently bind one or more mammalian chemokine receptor(s).
18. A method of identifying substances that may bind the protein molecules encoded within those in 1.
19. A method of identifying agents that modulate the transcription of the nucleic acids herein encoded within those in 1.
20. A method of identifying agents that modulate the translation of protein(s) derived from those in 8.
21. A method of identifying agents that modulate post-translational modification of protein(s) derived from those in 8.
22. A composition for the treatment of inflammation in mammalian organisms composed of the nucleic acids in 1.
23. A composition for the treatment of inflammation in mammalian organisms, where said composition includes the protein(s) in 8.
24. A composition for the treatment of human disorders mediated by inflammation, where said composition includes the nucleic acids in 1.
25. A composition for the treatment of human disorders mediated by inflammation, where said composition includes the protein(s) in 8.
26. A composition for the treatment of human disorders mediated by autoimmunity, where said composition includes the nucleic acids in 1.
27. A composition for the treatment of human disorders mediated by autoimmunity, where said composition includes the protein(s) in 8.
28. A composition for the prevention of human disorders associated with inflammation, where said composition includes the nucleic acids in 1.
29. A composition for the prevention of human disorders associated with inflammation, where said composition includes the protein(s) in 8.
30. A composition for the prevention of human disorders associated with autoimmunity, where said composition includes the nucleic acids in 1.
31. A composition for the prevention of human disorders associated with autoimmunity, where said composition includes the protein(s) in 8.
32. A composition for the treatment of a human disorder, where said disorder is either:
   (i) allergic inflammation; or
   (ii) asthma; or
   (iii) psoriasis; or
   (iv) diabetes mellitus; or
   (v) rheumatoid arthritis; or
   (vi) multiple sclerosis; or
   (vii) lupus erythmateous; or
   (viii) transplant rejection;
   (ix) graft rejection; or
   (x) glomerulonephritis; or
   (xi) arterial restenosis; or
   (xii) coronary occlusion; or
   (xiii) transplant vasculopathy; or
   (xiv) atherosclerosis; or
   (xv) unstable atherosclerotic plaque rupture; or
   (xvi) Ischemic reperfusion injury; or
   (xvii) glomerulonephritis.
33. As in 32, where said disorder is of a chronic nature.
34. As in 32, where said disorder is of an acute or sub-acute nature.
35. A method of treating a mammal having an immunomodulatory disorder, said method comprising administering to a mammal a therapeutically effective amount of a compound of 1 or 8, wherein said compound has an immunomodulatory effect in said mammal.
36. The method of 35, wherein the disorder is selected from allergic inflammation, asthma, psoriasis, diabetes mellitus, rheumatoid arthritis, multiple sclerosis, lupus, erythmateous, transplant rejection, graft rejection, glomerulonephritis, arterial restenosis, coronary occlusion, transplant vasculopathy, atherosclerosis, unstable atherosclerotic plaque rupture, ischemic reperfusion injury, and glomerulonephritis.
37. A substantially pure Yatapoxvirus cytoldne inhibitor polypeptide.
38. A substantially pure Yatapoxvirus nucleic acid molecule, wherein said nucleic acid molecule encodes a Yatapoxvirus cytokine inhibitor polypeptide.
39. The nucleic acid molecule of claim 11, wherein said nucleic acid molecule is selected from genomic DNA, cDNA, and mRNA.
40. A vector comprising the nucleic acid molecule of 1 or 38.
41. The vector of 40, wherein said vector is a gene therapy vector.
42. A cell comprising the vector of 40.
43. The vector of 40, wherein said nucleic acid molecule is operably linked to regulatory sequences for expression of Yatapoxvirus cytokine inhibitor polypeptide and wherein said regulatory sequences comprise a promoter.
44. A non-human transgenic animal comprising the nucleic acid molecule of 1 or 38.
45. A cell from the non-human transgenic animal of 44.
46. A non-human transgenic animal having a knockout mutation in one or both alleles encoding a polypeptide substantially identical to a Yatapoxvirus cytokine inhibitor polypeptide.
47. A probe for analyzing a Yatapoxvirus gene or a Yatapoxvirus gene homolog or fragment thereof, said probe having at least 50% nucleotide sequence identity to a sequence encoding a Yatapoxvirus cytokine inhibitor polypeptide or fragment thereof, wherein said polypeptide fragment comprises at least six amino acids, and said probe hybridizes under high stringency conditions to at least a portion of a Yatapoxvirus nucleic acid molecule.
48. A kit for the analysis of a Yatapoxvirus nucleic acid molecule, said kit comprising a nucleic acid molecule probe for analyzing a Yatapoxvirus nucleic acid molecule present in a test subject
49. A kit for the analysis of a Yatapoxvirus cytokine inhibitor polypeptide, said kit comprising an antibody for analyzing a Yatapoxvirus cytokine inhibitor polypeptide present in a test subject.

## Claims

1. A therapeutic composition for the treatment of an inflammatory or immune-mediated disease condition, the therapeutic composition comprising an isolated Yatapoxvirus cytokine inhibitor protein encoded by the amino acid sequence of SEQ ID NO: 2 or a homolog, truncation or analog thereof, wherein said protein binds to a mammalian cytokine and wherein said protein is formulated in a pharmaceutically acceptable vehicle.

2. The therapeutic composition of claim 1, wherein said isolated Yatapoxvirus cytokine inhibitor protein consists of the amino acid sequence of SEQ ID NO: 2.

3. The therapeutic composition of claims 1 or 2, wherein said cytokine is selected from the group consisting of IL-1, IL-4, IL-6, IL-18, TNF-α, and IFN-gamma.

4. The therapeutic composition of claim 3, wherein said cytokine is IL-18.

5. The therapeutic composition of claims 1-4, wherein said Yatapoxvirus cytokine inhibitor protein prevents the interaction between said cytokine and its corresponding cytokine receptor.

6. A method of modulating cytokine function in a cell in vitro, said method comprising contacting said cell with an isolated Yatapoxvirus cytokine inhibitor protein encoded by the amino acid sequence of SEQ ID NO: 2 or a homolog, truncation or analog thereof, wherein said protein non-covalently binds one or more mammalian cytokine(s).

7. The method of claim 6, wherein said isolated Yatapoxvirus cytokine inhibitor protein consists of the amino acid sequence of SEQ ID NO: 2.

8. The method of claims 6 or 7, wherein said cytokine is selected from the group consisting of IL-1, IL-4, IL-6, IL-18, TNF-α, and IFN-gamma.

9. The method of claim 8, wherein said cytokine is IL-18.

10. The method of claims 6-9, wherein said Yatapoxvirus cytokine inhibitor protein prevents the interaction between said cytokine and its corresponding cytokine receptor.

11. Use of an isolated Yatapoxvirus cytokine inhibitor protein encoded by the amino acid sequence of SEQ ID NO: 2 or a homolog, truncation or analog thereof, wherein said protein binds to a mammalian cytokine for the manufacture of a medicament for the treatment of an inflammatory or immune-mediated disease condition.

12. The use of claim 11, wherein the medicament is further **characterized** as defined any of claims 2 to 5.

13. A protein molecule encoded by a nucleic acid molecule derived from the Yaba Monkey Tumor Virus, as defined by SEQ ID NO:1, or fragments, analogs and homologs thereof, or as encoded by SEQ ID NO:2.

14. A method of modulating chemokine function through interaction between said proteins according to claim 16, wherein said proteins non-covalently bind one or more mammalian chemokine(s).

15. A method of modulating cytokine function through interaction between said proteins according to claim 16, wherein said proteins covalently bind one or more mammalian cytokine(s).

16. A method of modulating chemokine receptor function through interaction between said proteins according to claim 16, wherein said proteins non-covalently bind one or more mammalian chemokine receptor(s).

17. A composition for the treatment of inflammation in mammalian organisms, where said composition includes the protein(s) as claimed in claim 16.

18. A composition for the treatment of human disorders mediated by inflammation, where said composition includes the protein(s) as claimed in claim 16.

19. A composition for the treatment of human disorders mediated by autoimmunity, where said composition includes the protein(s) as claimed in claim 16.

20. A composition for the prevention of human disorders associated with inflammation, where said composition includes the protein(s) as claimed in claim 16.

21. A composition for the prevention of human disorders associated with autoimmunity, where said composition includes the protein(s) as claimed in claim 16.

22. A composition for the treatment of a human disorder, where said disorder is either:
(i) allergic inflammation; or
(ii) asthma; or
(iii) psoriasis; or
(iv) diabetes mellitus; or
(v) rheumatoid arthritis; or
(vi) multiple sclerosis; or
(vii) lupus erythmateous; or
(viii) transplant rejection;
(ix) graft rejection; or
(x) glomerulonephritis; or
(xi) arterial restenosis; or
(xii) coronary occlusion; or
(xiii) transplant vasculopathy; or
(xiv) atherosclerosis; or
(xv) unstable atherosclerotic plaque rupture; or
(xvi) ischemic reperfusion injury;

23. A method of treating a mammal having an immunomodulatory disorder, said method comprising administering to a mammal a therapeutically effective amount of a compound of claim 16, wherein said compound has an immunomodulatory effect in said mammal.

24. A substantially pure Yatapoxvirus cytokine inhibitor polypeptide.

25. Any product, method or use disclosed in the description including examples and figures.


SEQUENCE LISTING

<110> Viron Therapeutics Inc.

<120> Therapeutic composition comprising isolated Yatapoxvirus cytokine
inhibitor and uses thereof

<130> C62250PCEPT1

<150> Divisional of EP 01999573.7
<151> 2001-12-04

<150> US 60/251,147
<151> 2000-12-04

<160> 2

<170> FastSEQ for Windows Version 4.0

<210> 1
<211> 411
<212> DNA
<213> Yaba Monkey Tumor Virus

<400> 1
atgaaaaaaa ttgcaattat tttgtttttg ttgagttttt gtttttcatg tgacggtgaa 60
aaagaatgcg ataagcatag aagcgttaat attcaagttc cgatgaaaga aactagcgag 120
gtgttgttaa ggtgtaccgg tagttcgtat tttaagcatt ttagttatgt ttactggctt 180
gtgggagaaa gcgaaaccgt agatcagttg caacaaaatt ccggatatgg tgaaaccagt 240
cacccttcaa aacctcacga gtgtggaaac ttacctagcg ccgatttagt tctgacgaat 300
atgacagaaa aaatgcgtga cacaaagttg acttgtgtgt taatggaccc agacggacac 360
attgacgaat ctttagtatt acgcgaagtg tgggattgtt ttaacaaaac a 411

<210> 2
<211> 137
<212> PRT
<213> Yaba Monkey Tumor Virus

<400> 2
Met Lys Lys Ile Ala Ile Ile Leu Phe Leu Leu Ser Phe Cys Phe Ser
1               5                   10                  15
Cys Asp Gly Glu Lys Glu Cys Asp Lys His Arg Ser Val Asn Ile Gln
            20                  25                  30
Val Pro Met Lys Glu Thr Ser Glu Val Leu Leu Arg Cys Thr Gly Ser
        35                  40                  45
Ser Tyr Phe Lys His Phe Ser Tyr Val Tyr Trp Leu Val Gly Glu Ser
    50                  55                  60
Glu Thr Val Asp Gln Leu Gln Gln Asn Ser Gly Tyr Gly Glu Thr Ser
65                  70                  75                  80
His Pro Ser Lys Pro His Glu Cys Gly Asn Leu Pro Ser Ala Asp Leu
                85                  90                  95
Val Leu Thr Asn Met Thr Glu Lys Met Arg Asp Thr Lys Leu Thr Cys
            100                 105                 110
Val Leu Met Asp Pro Asp Gly His Ile Asp Glu Ser Leu Val Leu Arg
        115                 120                 125
Glu Val Trp Asp Cys Phe Asn Lys Thr
    130                 135